# EUROPEAN PATENT APPLICATION

(11) **EP 1 550 728 A1**
(43) Date of publication of application: **06.07.2005**
(21) Application number: 03721030.9
(22) Date of filing: 06.05.2003
(51) Int. Cl.: C12P 21/00

(54) **LYOPHILIZED PREPARATION FOR SYNTHESIS OF CELL-FREE PROTEIN**

(30) Priority: 14.05.2002 JP 2002138828
(71) Applicant: CellFree Sciences Co., Ltd., Yokohama-shi, Kanagawa 230-0046 (JP)
(72) Inventor: ENDO, Yaeta, Matsuyama-shi, Ehime 791-8016 (JP); OGASAWARA, Tomio, Iyo-shi, Ehime 799-3104 (JP)
(74) Representative: De Clercq, Ann
(86) International application number: PCT/JP2003/005656
(87) International publication number: WO 2003/095661

(57) **Abstract**

Freeze-dried preparations from cell extract for use in cell-free protein, which exhibit an activity for protein synthesis being in no way inferior to that exhibited in low-temperature preservation; and a method of utilizing it. In particular, preparations are formed by performing a freeze drying after reducing the concentration of deliquescent substances in a solution containing cell extract for synthesis of cell-free protein to a level not detrimental to the quality of preparations after the freeze drying.

## Description

The present application claims a priority from Japanese patent application No. 2002/138,828 which is quoted here by reference.

### Technical Field

The present invention relates to a freeze-dried preparation containing a cell extract which is used for a cell-free protein synthesis, to a process for synthesis of cell-free protein using said preparation and to a kit for synthesis of cell-free protein comprising said preparation.

### Background of the Invention

Synthetic reaction of protein taking place in cells proceeds in such steps that, firstly, information from DNA having genetic information is transcribed to mRNA and ribosome translates the information of mRNA whereupon protein is synthesized. At present, as a method where the protein synthesis in cells is conducted *in vitro* such as in a test tube, there have been briskly carried out studies for a method where, for example, ribosome and other components necessary for the protein synthesis are extracted from living organisms (hereinafter, that may be referred to as a "cell extract for the cell-free protein synthesis") and are used for the synthesis of cell-free protein in vitro (Japanese Patent Laid-Open No. 06/098,790; Japanese Patent Laid-Open No. 06/225,783; Japanese Patent Laid-Open No. 07/000, 194; Japanese Patent Laid-Open No. 09/000,291; and Japanese Patent Laid-Open No. 07/147,992).

A solution containing such the cell extract for the cell-free protein synthesis and a reaction solution for the protein synthesis to which components necessary for a translation reaction except enzymes and translation template are added to said solution containing the extract (hereinafter, that may be referred to as "solution containing the cell extract of the ready-made type") are unstable at room temperature and are able to be preserved only at extreme cold as not higher than -80°C. However, a cell-free protein synthetic system is a useful method where correctness and speed of the translation reaction maintain properties which are comparable to those of living cells and, in addition, aimed protein is able to be prepared without carrying out complicated purifying steps. Therefore, for more application of said synthetic system to industry, it is necessary not only that synthetic efficiency is enhanced but also that the above-mentioned solution containing the cell extract for synthesis and solution containing the cell extract of the ready-made type are provided in high quality in a constantly stable manner. Up to now, for the cell-free protein synthetic reaction, it has been necessary that each of various components such as amino acids, ATP, GTP, potassium and magnesium in addition to the solution containing cell extract is held in a stable state and that, prior to the initiation of the protein synthesis reaction, they are mixed at the optimum ratio. In the method as such, troublesome operations are needed in addition to the preservation method and, therefore, big problems are resulted when a synthetic sample is in large quantities. Further, the conventional method as such is a neck for construction of a totally-automated system for a high-throughput protein synthesis.

The present inventors have previously proposed a method where a solution containing the cell extract for the synthesis of protein and the solution containing the cell extract of the ready-made type are made into a preparation by a freeze drying (Japanese Patent Laid-Open No. 2000/316,594). However in said preparation, dissolution or the like happens during the freeze drying step whereby there is a problem that debasement in quality of said preparation takes place. "Debasement in quality" means that, when water is added to said preparation, the preparation is not completely dissolved and a synthetic activity in the protein synthesis reaction using the same lowers as well.

### Summary of the Invention

In order to solve the above-mentioned problems, the present inventors have carried out intensive investigations. As a result, they have found that, when concentration of a deliquescent substance contained in the solution containing an extract for the cell-free protein synthesis such as potassium acetate is lowered to an extent of not higher than 60 mM followed by the freeze drying and making into a preparation, it is now possible to maintain said solution in such a stable manner that it is able to be conducted in industry whereupon the present invention has been achieved.

Thus, the present invention provides the followings.
1. A method for the manufacture of a freeze-dried preparation for a cell-free protein synthesis by a freeze drying of a solution containing a cell extract for a cell-free protein synthesis, wherein the content of the deliquescent substance contained in the solution is in such an amount that it does not affect the quality of the preparation after being freeze-dried.
2. A freeze-dried preparation for the cell-free protein synthesis according to the above 1, wherein the amount of the deliquescent substance is not more than 0.01 part by weight to 1 part by weight of the protein in the dried preparation.
3. The method according to the above 1 or 2, wherein the deliquescent substance is selected from potassium acetate, magnesium acetate and calcium acetate.
4. The method according to the above 1, wherein the method includes a step where a low-molecular inhibitor for the protein synthesis is excluded from the solution containing the cell extract for the cell-free protein synthesis.
5. The method according to the above 4, wherein the low-molecular inhibitor for the protein synthesis has a molecular weight of not more than 14,000 daltons.
6. The method according to the above 4 or 5, wherein exclusion of the inhibitor for the protein synthesis is carried out in a solution which at least contains a high-energy phosphoric acid compound.
7. The method according to any of the above 1 to 6, wherein the solution containing a cell extract for a cell-free protein synthesis is an extract of wheat germ.
8. The method according to any of the above 1 to 7, wherein the solution containing the cell extract for the cell-free protein synthesis is that to which a solution containing amino acid which is a substance necessary for the protein synthesis at least acting as a substrate therefor and energy source are contained is added.
9. A freeze-dried preparation for a cell-free protein synthesis which is manufactured by the method mentioned in the above 1 to 8.
10. A kit for a cell-free protein synthesis containing the preparation mentioned in the above 9.
11. A kit for a cell-free protein synthesis in which the preparation mentioned in the above 9 is combined with a solution so that concentration of a solution of at least the deliquescent substance is adjusted to optimum during the cell-free protein synthesis.
12. The kit for the protein synthesis according to the above 10 or 11, wherein a protein library is able to be synthesized by way of containing a translation template.
13. A method for the protein synthesis in which a solution containing a cell extract for a cell-free protein synthesis which is prepared using the kit for the cell-free protein synthesis mentioned in the above 10 to 12 is used.
14. The method for the protein synthesis according to the above 13, wherein a preparation is conducted so as to make a final concentration of potassium acetate 50 to 200 mM.
15. A synthetic protein which is prepared by the method for the protein synthesis mentioned in the above 13.

### Brief Description of the Drawings

Fig. 1 is a graph which shows the cell-free protein synthesis activity using the freeze-dried preparation of a solution containing the wheat germ extract where concentrations of potassium acetate are different.
Fig. 2 is a graph which shows the cell-free protein synthesis activity by the solution containing the wheat germ extract subjected to a treatment for exclusion of a low-molecular substance in which concentrations of potassium acetate are changed.

### Detailed Description of the Invention

The present invention will be illustrated in more detail as hereunder.

The freeze-dried preparation of the present invention is able to be manufactured when concentration of a deliquescent substance contained in a solution containing the cell extract for the cell-free protein synthesis is reduced to an extent of not affecting on the quality of the preparation after being freeze-dried and then it is freeze-dried. The solution containing the cell extract for the cell-free protein synthesis which is able to be used for the manufacture of the freeze-dried preparation of the present invention contains not only that where necessary components for the protein synthesis is extracted from cells but also that of the ready-made type in which substrates necessary for the cell-free protein synthesis system such as various ions and amino acids, energy source, buffer and other effective factors are added to the cell extract-containing solution. Method for the preparation of each of them will be illustrated in detail as hereunder.

### (1) Solution containing the cell extract for the cell-free protein synthesis

### [Solution containing the cell extract]

With regard to the solution containing the cell extract for the cell-free protein synthesis used for the present invention, anything may be used so far as it has a protein synthetic ability in the cell-free protein synthesis system. Here, the cell-free protein synthesis system means a method which is conducted in vitro where a component containing such as ribosome which is a protein translation device installed in cells is extracted from living organism and then transcription or translation template, nucleic acid and amino acid which are to be substrates, energy source, various ions, buffer and other effective factor are added to the extract. In the above, there are a case where RNA is used as a template (hereinafter, that may be referred to as a "cell-free translation system") and a case where DNA is used and enzyme necessary for transcription such as RNA polymerase are further added thereto followed by conducting the reaction (hereinafter, that may be referred to as a "cell-free transcription/translation system"). The cell-free protein synthesis system in the present invention covers both of the above-mentioned cell-free translation system and the cell-free transcription/translation system.

To be more specific, with regard to the cell extract used in the present invention, that which is prepared from microbe such as *Escherichia coli,* germs of plant seeds, reticulocytes of mammals such as rabbit, etc. may be used. With regard to the solution containing the cell extract, that which has been commercially available may be used and that may be prepared by a known method *per se* or, to be more specific, a cell extract of microbes such as *Escherichia coli* may be prepared according to a method mentioned, for example, in Pratt, J. M. et al., Transcription and Translation, Hames, 179-209, B. D. & Higgins, S. J., eds., IRL Press, Oxford (1984).

With regard to a commercially available cell extract, *E. coli* S30 Extract System (manufactured by Promega) and RTS 500 Rapid Translation System (manufactured by Roche) are exemplified as that derived from *Escherichia coli;* Rabbit Reticulocyte Lysate System (manufactured by Promega) , etc. are exemplified as that derived from reticulocytes of rabbit; and PROTEIOS™ (manufactured by Toyobo), etc. are exemplified as that derived from wheat germs. Among them, it is preferred to use a germ extract of plant seeds and, with regard to the plant seeds, those of plants of *Gramineae* such as wheat, barley, rice and corn are preferred. With regard to the cell extract, that using the wheat germ extract of is suitable among them.

With regard to a method for preparing the wheat germ extract, a method mentioned, for example, in Johnston, B. F. et al., Nature, 179, 160-161 (1957), etc. is used as a method for isolation of wheat germs while, with regard to a method for extracting a cell extract from isolated germs, a method mentioned, for example, in Erickson, A. H. et al. (1996) *Meth. in Enzymol.,* 96, 38-50 may be used and illustrations therefor will be given in detail as hereunder.

Since the amount of germs contained in plant seeds is little, it is preferred for an efficient production of germs to remove areas other than germs as much as possible. It is usual that, firstly, mechanical force is applied to plant seeds to prepare a mixture containing germs, disintegrated endosperms, disintegrated seed coats, etc. and then disintegrated endosperms, disintegrated seed coats, etc. are removed from said mixture whereupon a crude germ fraction (a mixture where germ is a main component containing disintegrated endosperms and disintegrated seed coats) is prepared. The force to be applied to plant seeds may be in such an extent that it is able to separate germs from plant seeds.

Usually, plant seeds are disintegrated using a known disintegrating apparatus to give a mixture containing germs, disintegrated endosperms and disintegrated seed coats.

Disintegration of plant seeds is usually carried out using a known disintegrating apparatus and it is preferred to use a disintegrating apparatus of a type where impulsive force is applied to a thing to be disintegrated such as pin mill and hammer mill. Degree of disintegration may be appropriately selected depending upon the size of plant seed germs to be used and, in the case of wheat seeds for example, it is disintegrated usually into a size where the maximum size is not bigger than 4 mm or, preferably, not bigger than 2 mm. It is preferred that the disintegration is conducted by a dry method.

After that, a crude germ fraction is prepared from the resulting disintegrated plant seeds using, usually, a known sieving apparatus such as a sieve. In the case of wheat seeds for example, the crude germ fraction of usually in a mesh size of 0.5 mm to 2.0 mm or, preferably, 0.7 mm to 1.4 mm is prepared. If necessary, seed coats, endosperms, dust, etc. contained in the resulting crude germ fraction may be removed by means of wind force or isoelectric force.

It is also possible to prepare the crude germ fraction by a method where difference between germ and seed coat/endosperm is utilized such as a heavy-media separation. For a purpose of preparation of a crude germ fraction containing more germs, two or more of the above-mentioned methods may be combined. Further, germs are selected from the resulting crude germ fraction by means of naked eye, color selector or the like.

There are some cases where endosperm components are adhered to the germ fraction prepared as such and, therefore, it is usually preferred to subject to a further washing treatment for purification of germs.

With regard to a washing treatment, it is usually preferred that the germ fraction is dispersed/suspended in water or an aqueous solution which is cooled at not higher than 10°C or, preferably, not higher than 4°C and then washing is conducted until the washing is no longer turbid. It is usually more preferred that the germ fraction is dispersed/suspended in an aqueous solution of a surfactant at not higher than 10°C or, preferably, not higher than 4°C and then washing is conducted until the washing is no longer turbid. With regard to a surfactant, a nonionic one is preferred and that may be widely utilized so far as it is a nonionic surfactant. To be more specific, preferred examples thereof are polyoxyethylene derivatives such as Brij, Triton, Nonidet P40 and Tween. Among them, Nonidet P40 is most suitable. The nonionic surfactant as such may be used in a concentration of 0.5% for example.

With regard to the washing treatment using water or aqueous solution and the washing treatment using a surfactant, any of them may be used or both may be carried out. Further, such a washing treatment may be conducted in combination with an ultrasonic treatment.

After plant germs are selected from the disintegrated product which is prepared by disintegration of plant seeds as mentioned above, undamaged germs (having a germinating ability) prepared by washing are finely divided, finely divided plant germs and an extracting solvent are mixed to prepare a germ extract and a solution containing a germ extract is separated and purified.

Fine division of plant germs in the present invention may be carried out by grinding or disintegrating with the pressure of the frozen germ using a mortar or the like as in the conventional manner and it is preferred to conduct a fine division by means of impact or cutting as previously proposed by the present inventors (Japanese Patent Application No. 2002/023, 139 and Japanese Patent Application No. 2002/231,340). Here the term "to conduct a fine division by means of impact or cutting" used here means that plant germs are destructed under such a condition that destruction of organelle such as cellular nucleus of plant germs, mitochondria and chlorophyll, cell membrane, cell wall, etc. is able to be kept in minimum as compared with the conventional grinding or disintegration with pressure.

With regard to apparatus and method which are able to be used for the fine division, although there is no particular limitation so far as it satisfies the above-mentioned conditions, it is preferred to use an apparatus equipped with a rotating edge in a high speed such as a Waring blender (as hereunder, such a method may be just referred to as "a Waring blender method"). Revolutions of the edge are usually not less than 1,000 rpm and, preferably, not less than 5,000 rpm. Further, they are usually not more than 30,000 rpm and, preferably, not more than 25, 000 rpm. Rotating time of the edge is usually not shorter than 5 seconds and, preferably, not shorter than 10 seconds. Although there is no particular limitation for the rotating time, it is usually not longer than 10 minutes and, preferably, not longer than 5 minutes. Although there is no particular limitation for temperature for the fine division so far as the synthetic ability of germ is not lost, it is preferably not higher than 10°C and a temperature range by which an operation for the fine division is possible, particularly preferably about 4°C, is appropriate.

When germs are subjected to the fine division by impact or cutting as in the present invention, all of organelles such as cell nucleus of the germ, cell membrane and cell wall are not destructed and at least a part thereof is not destructed but remains. Thus, organelles such as cell nucleus of the germ, cell membrane and cell wall are not unnecessarily destructed and, therefore, impurity contamination with impurities such as DNA and lipid is little whereby it is now possible that RNA, ribosome, etc. being locally present in cytoplasm which are necessary for the protein synthesis are able to be efficiently extracted in a high purity from germs.

After that, an extract is added to finely divided germs followed by stirring, the solution containing the germ extract is recovered by, for example, means of a centrifugal separation and purified by, for example, means of a gel filtration whereupon the germ extract is able to be prepared.

When the extracting solvent is present during the fine division of germs by impact or cutting, it is possible that a step of fine division of plant germs and a step of preparing a germ extract by mixing finely divided plant germs and the extracting solvent are able to be carried out at the same time. In that case, germs and the extracting solvent in an amount of being necessary for the extraction are mixed and germs are finely divided in the presence of the extracting solvent. Amount of the extracting solvent to 1 g of germs before washing is usually not less than 0.1 ml, preferably not less than 0.5 ml and, more preferably, not less than 1 ml. Although there is no particular limitation for the upper limit of the amount of the extracting solvent, it is usually not more than 10 ml and, preferably, not more than 5 ml to 1 g of germs before washing. With regard to germs to be finely divided, although those which are frozen as in the conventional art may be used or those which are not frozen may be used, it is preferred to use germs which are not frozen.

With regard to an extracting solvent, it is possible to use an aqueous solution containing buffer, potassium ion, magnesium ion and/or antioxidant for thiol group. If necessary, calcium ion, L-amino acid, etc. may be further added thereto. For example, it is possible to use a solution containing N-hydroxyethylpiperazine-N'-2-ethanesulfonic acid (HEPES)-KOH, potassium acetate, magnesium acetate, L-amino acid and/or dithiothreitol or a solution where a method of Patterson, et al. is partially modified (a solution containing HEPES-KOH, potassium, magnesium acetate, calcium chloride, L-amino acid and/or dithiothreitol) as the extracting solvent. Composition and concentration of each component in the extracting solvent are known *per se* and that which has been commonly used for the method for the manufacture of the germ extract for the cell-free protein synthesis may be adopted.

After that, the solution containing the germ extract is recovered by, for example, centrifugal separation and purified by, for example, the gel filtration whereupon the germ extract is able to be prepared. With regard to the gel filtration, it is able to be conducted using, for example, a gel filtration apparatus such as Sephadex G25 which is previously equilibrated with a solution (s solvent containing HEPES-KOH, potassium acetate, magnesium acetate, dithiothreitol or L-amino acid). Composition and concentration of each component in the gel filtration solution may be known *per se* and those which have been commonly used for the method for the manufacture of the germ extract for the cell-free protein synthesis may be adopted.

In the germ extract prepared as such, endosperm containing substances which suppress the protein synthesis function (substances such as tritin, thionine, ribonuclease, etc. which act on mRNA, tRNA, translation protein factor, ribosome, etc.) being contained in or held by material cells themselves is removed almost completely and is in a pure state. Here, the expression that endosperm is removed almost completely means that the germ extract where an endosperm part is removed to such an extent that ribosome is not substantially deadenylated. The expression that to such an extent that ribosome is not substantially deadenylated means that the deadenylating rate of ribosome is less than 7% or, preferably, not more than 1%.

The above-mentioned germ extract contains protein being derived from the cell extract (and that being added separately upon necessity). Although there is no particular limitation for the amount thereof, it is preferably 1 to 10% by weight or, more preferably, 2.5 to 5% by weight in the total amount of the said composition in the case of a composition before the freeze drying while it is preferably 10 to 90% by weight or, more preferably, 25 to 70% by weight in the total amount of the said composition in a composition after freeze drying in view of stability in the freeze-dried state upon preservation and of easy use. If necessary, the amount of the protein may be made within the above-mentioned range in the composition before the freeze drying by a common method such as the gel filtration and a dialysis.

Incidentally, a protein content mentioned hereinabove is calculated by measurement of the absorbance (260, 280 and 320 nm).

### [Exclusion of low-molecular substances which inhibit the synthesis]

The cell extract as such further contains low-molecular substances which inhibit the synthesis having a suppressive activity for protein synthesis. As the present inventors have already proposed, a high protein synthesis activity is able to be maintained even after making into a freeze-dried preparation when such inhibitors are excluded (patent application pending). To be more specific, such low-molecular synthesis inhibitors are fractionated and excluded from a constituent of the cell extract by means of difference in molecular weights. Molecular weights of substances to be excluded (low-molecular inhibitors for the protein synthesis) may be less than the factor necessary for the protein synthesis contained in the cell extract. To be more specific, those having molecular weight of 50,000 to 14,000 and below, preferably, not more than 14,000 may be exemplified.

With regard to a method for exclusion of the low-molecular substance for inhibiting the synthesis, a commonly used method which has been known *per se* is used and, to be more specific, a method by the dialysis using a dialysis membrane, a gel filtration method and an ultrafiltration method may be exemplified. Among them, a method by the dialysis (a dialysis method) is preferred in view of easy supply of substances to the inner liquid for the dialysis. As hereunder, detailed illustration will be given taking the case where the dialysis method is used as an example.

With regard to the dialysis membrane used for the dialysis, that having an exclusion molecular weight of 50,000 to 12, 000 is exemplified. To be more specific, a regenerated cellulose membrane having an exclusion molecular weight of 12,000 to 14, 000 (manufactured by Viskase Sales, Chicago), Spectra/Pore 6 (manufactured by Spectrum Laboratories, Inc., CA, U. S. A.) having an exclusion molecular weight of 50,000, etc. may be preferably used. The above-mentioned cell extract in an appropriate amount is placed in such the dialysis membrane and the dialysis is carried out by a common method. Time for conducting the dialysis is preferably about 30 minutes to 24 hours.

When an insoluble component is produced in the cell extract in case exclusion of low-molecular synthesis inhibitors is carried out as such, that is inhibited (hereinafter, it may be referred to as a "stabilization of the cell extract") whereupon protein synthesis activity of a finally produced cell extract (hereinafter, it may be referred to as a "cell extract after the treatment") is enhanced. With regard to a specific method for stabilization of the cell extract, a method where the exclusion is carried out in a solution containing at least a high-energy phosphoric acid compound such as ATP or GTP when exclusion of the low-molecular inhibitor for the protein synthesis mentioned in the above (1) is conducted is exemplified. With regard to the high-energy phosphoric acid compound, ATP is preferably used. It is carried out in a solution preferably containing ATP and GTP or, more preferably, containing ATP, GTP and twenty kinds of amino acids.

When exclusion of low-molecular inhibitors for the protein synthesis is conducted in a solution containing the above-mentioned components (hereinafter, they may be referred to as "stabilizing components"), it is also possible that stabilizing components are previously added to the cell extract followed by subjecting to incubation and are used for an exclusion step for low-molecular inhibitors for the protein synthesis. When the dialysis method is used for exclusion of low-molecular synthesis inhibitors, it is also possible that stabilizing substances are added not only to the cell extract but also the outer liquid for the dialysis and the dialysis is conducted to exclude low-molecular inhibitors for the protein synthesis. When stabilizing components are also added to the outer liquid for the dialysis as such, new stabilizing components are supplied at all times even when stabilizing components are degraded during the dialysis and that is more preferred. That is also applicable to the case where the gel filtration method or an ultrafiltration method is used. The same effect is able to be achieved when the process is conducted in such a manner that each carrier is equilibrated with a buffer for filtration containing stabilizing components, then the cell extract containing stabilizing components is supplied and filtration is carried out together with addition of the above-mentioned buffer.

With regard to adding amount of stabilizing components and time for the stabilizing treatment, they may be appropriately selected depending upon the type and the preparation method for the cell extract. With regard to a method for the selection therefor, an example is a method where stabilizing components to which amount and type are tentatively assigned are added to the cell extract, an exclusion step for low-molecular inhibitors for the protein synthesis is conducted after an appropriate time, the resulting cell extract after the treatment is separated into soluble and insoluble components by a method such as centrifugal separation and the part containing less amount of insoluble components is selected. A method where the cell-free protein synthesis is carried out using the resulting cell extract after the treatment and a part showing a high protein synthesis activity is selected is preferred as well. Further, when the cell extract and the dialysis method are used in the above-mentioned method for the selection, there is also a method where an appropriate stabilizer is also added to the outer liquid for the dialysis, the dialysis is conducted for an appropriate time using that and then selection is carried out depending upon the amount of the insoluble component in the resulting cell extract, the protein synthesis activity of the resulting cell extract, etc.

A specific example of the stabilizing condition for the cell extract selected as such in case an exclusion step of low-molecular inhibitors for the protein synthesis by the dialysis method is conducted using the wheat germ extract prepared by a blender method mentioned in (1) is a method where 100 µM to 0.5 mM of ATP, 25 µM to 1 mM of GTP and 25 µM to 5 mM of each of the twenty kinds of amino acids are added to the wheat germ extract and the outer liquid for the dialysis and then the dialysis is carried out for not shorter than 30 minutes to 1 hour. Any temperature may be applied for conducting the dialysis so far as protein synthesis activity is not lost and the dialysis is possible. To be more specific, the lowest temperature is a temperature whereby the solution is not frozen which is usually -10°C or, preferably, -5°C while, the highest temperature is a temperature limit whereby the solution used for the dialysis is not affected which is 40°C or, preferably, 38°C.

There is no particular limitation for the method of addition of stabilizing components to the cell extract. Thus, it is possible that they are added before the exclusion step for low-molecular inhibitors for the protein synthesis, the mixture is incubated for an appropriate time to stabilize and then an exclusion step for low-molecular synthesis inhibitors is carried out or it is also possible that an exclusion step for low-molecular synthesis inhibitors is conducted using the cell extract to which stabilizing components are added and/or a buffer to be used for the exclusion step to which stabilizing components are added.

### [Weakly reduced cell extract for the cell-free protein synthesis]

When concentration of a reducing agent in a translation reaction solution is adjusted in the cell-free protein synthesis system as the present inventors have proposed already, it is possible to synthesize protein by maintaining a disulfide bond in a molecule (Japanese Patent Application No. 2002/053,161). The freeze-dried preparation of the present invention for preparing such the translation reaction solution is able to be prepared by adjusting the concentration of the reducing agent in the solution containing the cell extract. With regard to the specific reducing agent and its concentration, the range whereby the final concentration of dithiothreitol (hereinafter, it may be referred to as "DTT") in the translation reaction using said preparation is 20 to 70 µM or, preferably, 30 to 50 µM, the final concentration of 2-mercaptoethanol therein is 0.1 to 0.2 mM and the concentration of glutathione/reduced-type glutathione therein is made 30 to 50 µM/1 to 5 µM may be exemplified.

Concentration of the reducing agent in the translation reaction solution as above is not limited to the above-mentioned ones only but may be appropriately modified depending upon the type of protein to be synthesized or of cell-free protein system used. There is no particular limitation for a method of selecting the optimum concentration range for a reducing agent. However, an example is a method where judgment is done by the effect of enzyme which catalyzes the exchange reaction for the disulfide bond. To be more specific, translation reaction solutions to which various concentrations of the reducing agent are assigned and prepared, an enzyme which catalyzes the disulfide bond exchange reaction is added thereto and synthesis of protein having a disulfide in a molecule is carried out. As a control experiment, the same protein synthesis is carried out without addition of an enzyme which catalyzes the disulfide bond exchange reaction in the same translation reaction solution. Solubilized components of the protein synthesized here are separated by, for example, means of the centrifugal separation. A reaction solution in which amount of the solubilized components of protein synthesized here is not less than 50% of the whole (solubilization rate: 50%) and the solubilized components increased by addition of the enzyme which catalyzes the disulfide bond exchange reaction may be judged to be suitable as a reaction for the synthesis where the intramolecular disulfide bond in said protein is still maintained. Further, among the concentration ranges of a reducing agent selected by the effect of the enzyme which catalyzes the above disulfide bond exchange reaction, concentration of the reducing agent where a mass of the synthesized protein is largest is able to be selected as a more preferred concentration range.

With regard to a method for preparing a reaction solution containing the reducing agent of such a concentration, there may be used a method where the cell extract for the cell-free protein synthesis containing no reducing agent is prepared and then the reducing agent so as to give the above concentration range is added together with components necessary for the cell-free protein synthesis system, a method where the reducing agent is removed from the cell extract for the cell-free protein synthesis so as to give the above-mentioned concentration range, etc. In the case of the cell extract for the cell-free protein synthesis, a high reducing condition is necessary for the extraction and, therefore, a method where a reducing agent is removed from the solution after the extraction is easier. With regard to a method of removing the reducing agent from the cell extract, a method using a carrier for gel filtration, etc. may be exemplified. To be more specific, an example is a method where a Sephadex G-25 column is previously equilibrated with an appropriate buffer containing no reducing agent and then the cell extract is passed therethrough.

In case synthesis of protein is carried out using a preparation which is prepared by the freeze drying of a weakly reduced cell extract for the cell-free protein synthesis prepared as such, it is possible to synthesize protein in which intramolecular disulfide bond is maintained in a high efficiency when an enzyme which catalyzes the disulfide bond exchange reaction is added and followed by subjecting to the translation reaction. With regard to an enzyme which catalyzes the disulfide bond exchange reaction, a protein disulfide isomerase is exemplified. Adding amount of such an enzyme to the cell-free translation system may be appropriately selected depending upon the type of the enzyme. To be more specific, in the case of the cell extract for the cell-free protein synthesis extracted from wheat germs where the protein disulfide isomerase is added to the translation reaction solution containing 20 to 70 or, preferably, 30 to 50 µM of DDT as a reducing agent, it is added so as to make the final concentration within a range of 0.01 to 10 µM or, preferably, 0.5 µM. With regard to the stage when it is added, it is preferred to add before initiation of the cell-free translation reaction in view of efficiency of formation of a disulfide bond.

### [Solution containing the cell extract of the ready-made type]

The solutions containing various cell extracts prepared as such are able to carry out the protein synthesis when translation template, nuclease inhibitor, various ions, substrate, energy source, etc. necessary for the protein synthesis (hereinafter, they may be referred to as "additives for the translation reaction") are added and subjected to an appropriate system for the protein synthesis. Accordingly, the solution containing the cell extract for the cell-free protein synthesis for the manufacture of a freeze-dried preparation of the present invention also includes a solution to which additives for the translation reaction other than translation template and nuclease inhibitor are added to the above-mentioned various kinds of cell extracts (in the present invention, that may be referred to as "solution containing the cell extract of the ready-made type").

### (2) Manufacture of freeze-dried preparation

In the solution containing the cell extract mentioned in (1), an extracting solvent or a buffer used for gel filtration conducted after the extraction contains the deliquescent substance such as potassium acetate or magnesium acetate. Therefore, when a solution containing the cell extract prepared using the conventional extracting solvent is just prepared into a dry preparation, dissolution or the like takes place in the freeze drying step and, as a result, there is a problem that quality of said preparation is deteriorated. Deterioration of quality means that, when water is added to the said preparation, the preparation is not completely dissolved in water and the synthetic activity in a protein synthesis reaction using it is deteriorated as well.

Now, in the present invention, concentration of the deliquescent substance contained in the said solution containing the cell extract is decreased to such an extent that the substance does not affect the quality of the preparation. As hereunder, the solution containing the cell extract prepared here may be referred to as "the solution containing the cell extract where the deliquescent substance is decreased". Examples of the specific method for decreasing the deliquescent substance are the gel filtration method where a gel carrier which is previously equilibrated with a solution containing a decreased amount of or no deliquescent substance (hereinafter, the solution may be referred to as "a buffer where the deliquescent substance are decreased") is used, the dialysis method, etc. With regard to concentration of the deliquescent substance in the solution containing the cell extract, the total concentration thereof is decreased to 60 mM or lower. In the case of the solution containing the wheat germ extract, a concentration of potassium acetate contained in the extract is decreased to not more than 60 mM or, preferably, not more than 50 mM.

With regard to a substance having a deliquescent property (the deliquescent substance) in the above-mentioned freeze-dried preparation, its amount by which stability upon preservation in a freeze-dried state is not deteriorated is preferably not more than 0.01 part by weight and, particularly preferably, not more than 0.005 part by weight to 1 part by weight of protein in the said freeze-dried preparation. It is therefore preferred that, before the freeze drying, the cell extract is subjected to the gel filtration, dialysis, etc. so that the amount of said deliquescent substance in the freeze-dried composition is made within the above-mentioned range.

The above-mentioned decrease in the deliquescent substance may be carried out after the solution containing the cell extract is manufactured according to the method mentioned in (1) or may be carried out by applying the above-mentioned low-deliquescent buffer to the gel filtration or the dialysis contained in each manufacturing step.

In addition, the solution containing the cell extract where the deliquescent substance is decreased may be contaminated with spores of microbes, particularly, filamentous fungi (fungi) and it is preferred that such microbes are excluded. Growth of microbes are noted especially during a cell-free protein synthesis reaction for a long period (for not shorter than one day) and, therefore, it is important to inhibit the growth. Although there is no particular limitation for the excluding means for microbes, it is preferred to use a sterilization filter. With regard to pore size of the filter, there is no particular limitation so far as it is a size by which microbes having a possibility of being contaminated are able to be excluded and it is appropriate to be usually 0.1 to 1 micrometer or, preferably, 0.2 to 0.5 micrometer. Incidentally, spore size of *Bacillus subtilis* having a small size is 0.5 µm × 1 µm and, therefore, it is effective for removal of spores as well to use a filter of 0.20 micrometer (such as Minisart™ manufactured by Sartorius). In the filtration, it is preferred to filter firstly using a filter having big pore size and then using a filter having a pore size by which the microbes having a probability of contamination are able to be excluded.

When the solution containing the cell extract where the deliquescent substance is decreased prepared as such is freeze-dried, that is made into a preparation. Method for the freeze drying may be appropriately selected from commonly used methods which have been known *per se* and. To be more specific, there is used, for example, a method where the solution is quickly frozen by liquid nitrogen and then dried using a common freeze-dryer. It is preferred that freeze drying temperature, time, etc. are in accordance with those mentioned in the direction for use of the freeze-dryer used therefor. The freeze-dried preparation of the present invention which is prepared as a result of completion of removal of water is very stable at room temperature. And a solution containing the cell extract prepared by addition of water or the like thereto has a protein synthetic activity which is as good as that being preserved at extremely low temperature. It is further preferred as a method for preservation of said preparation to substitute air in the container for preservation with nitrogen gas.

### (3) Cell-free protein synthesis using the freeze-dried preparation for the cell-free protein synthesis

The translation reaction solution is prepared by addition of necessary water and component which is necessary for the translation reaction to the freeze-dried preparation for the cell-free protein synthesis of the present invention and then it is subjected to an appropriate cell-free protein synthesis system whereupon the cell-free protein synthesis is able to be carried out. The component to be added is a solution containing a substance by which the deliquescent substance in the translation reaction solution is adjusted to an optimum concentration. With regard to the substance and the amount in the said solution, a substance which has the preparation return to the state before conducting a step for decreasing the deliquescent substance for the freeze drying and a substance which is necessary to add for conducting the further translation reaction are exemplified. To be more specific, in the case of a preparation where the solution containing the cell extract for the cell-free protein synthesis where amount of potassium acetate as the deliquescent substance is decreased is freeze-dried, a potassium acetate solution or the like is added so that its final concentration in the solution containing the cell extract manufactured from the said preparation is adjusted to 50 to 200 mM.

When the deliquescent substance excluded in (2) is added in a necessary amount for a translation reaction to a solution which provides a component necessary for the translation reaction solution such as outer liquid for the dialysis or liquid in a lower vessel in the case of conducting the translation reaction by means of the dialysis method (Kigawa, et al., The 21st Meeting of Japan Molecular Biology Society, WID 6) or a double layer method (Sawasaki, T., et al., *FEBS Let.,* 514, 102-105 (2002)) which will be mentioned later, then it is not necessary to add thereto when a frozen preparation is returned.

When the translation reaction is carried out using the solution containing the cell extract mentioned in the above (1) "Solution containing the cell extract for the cell-free protein synthesis", concentration of the reducing agent is adjusted to the above-mentioned range.

With regard to the component which is necessary to be added for conducting the translation reaction, that is different between the case where a freeze-dried preparation is manufactured using the solution containing the cell extract of the ready-made type in (1) and the case where it is not. Anyway, it is preferred that, in the final translation reaction solution, each of amino acids acting as a substrate, energy source, various ions, buffer, ATP regeneration system, nuclease inhibitor, tRNA, reducing agent, polyethylene glycol, 3',5'-cAMP, folate, antibacterial agent, etc. is added to an extent of 100 µM to 0.5 mM as ATP, 25 µM to 1 mM as GTP and 25 µM to 5 mM as each of twenty kinds of amino acids.

When the wheat germ extract prepared by a blender method mentioned in the above (1) is used as the cell extract, it is usually unnecessary to add tRNA. When protein having the disulfide bond in the molecule is synthesized, it is preferred that concentration of the reducing agent is made within the range mentioned in the above (1).

Examples of a system or an apparatus for the protein synthesis are a method where energy source and amino acids necessary for the cell-free protein synthesis or tRNA are/is added to said cell extract as in a batch method (Pratt, J. M., et al., *Transcription and Translation,* Hames, 179-209, B. D. & Higgins, S. J., eds. IRL Press, Oxford (1984)), a continuous cell-free protein synthesis system where amino acids, energy source, etc. are continuously supplied to the reaction system (Spirin, A. S., et al., *Science,* 242, 1162-1164 (1988)), the dialysis method and the double layer method (Sawasaki, T., et al., *FEBS Let.,* 514, 102-105 (2002)). It is also possible to use a method where template RNA, amino acids, energy source, etc. are added upon necessity to the synthetic reaction system and the synthesized product and then the degraded product is discharged upon necessity (Japanese Patent Laid-Open No. 2000/333,673; hereinafter, it may be referred to as "discontinuous gel filtration method"), etc.

Since the translation reaction solution prepared as such contains the desired protein in a high concentration, the aimed protein is able to be easily prepared from the said reaction solution by a separation/purification method known *per se* such as the dialysis, the ion-exchange chromatography, an affinity chromatography or the gel filtration.

### (4) Kit for the cell-free protein synthesis

The freeze-dried preparation for the synthesis of protein according to the present invention is able to be provided as a kit for a cell-free protein synthesis containing at least said preparation. In addition to the said preparation, the kit of the present invention contains necessary components for preparing the translation reaction solution mentioned in (3), an RNA polymerase, a translation template nucleic acid for a positive control, vectors for preparing a translation template, buffers, reaction containers, etc.

When nucleic acid which is to be the translation template is contained therein, a kit for preparing a protein library which is a type of preparing the protein upon use is able to be provided. Further, when a fused product where nucleic acid coding for polypeptide specifically bonding to a certain substance is fused with nucleic acid coding for an aimed polypeptide and a solid phase substance bonding to the above-mentioned certain substance are added as the above-mentioned translation templates, a kit for preparing a protein chip of a type for preparing upon use is also able to be provided.

However, it is not necessary to contain all of those reagents but any combination of reagents may be used so far as it is a kit which is able to be used for the cell-free protein synthesis, etc. using the cell extract after the treatment of the present invention.

### Examples

The present invention will now be further illustrated by way of the following Examples although the following Examples are only to help for obtaining the specific recognition concerning the present invention and scope of the present invention is never limited to the following Examples.

### Example 1. Preparation of the solution containing the cell-free extract where the deliquescent substance is decreased and preparation of the freeze-dried preparation

### (1) Preparation of the wheat germ extract

Seeds (non-disinfected) of *chihoku* wheat produced in Hokkaido were added to a mill (Rotor Speed Mill Pulverisette, type 14; manufactured by Fritsch) at the rate of 100 g per minute and were gently ground at 8,000 rpm. After a fraction (mesh size: 0.7 to 1.00 mm) containing germs having a germinating capacity was recovered by a sieve, it was subjected to a floating using a mixed liquid of carbon tetrachloride and cyclohexane (ratio by volume: carbon tetrachloride : cyclohexane = 2.4 : 1) to recover a floated fraction containing germs having the germinating capacity, then the organic solvents were removed by drying at room temperature and contaminated impurities such as seed coats were removed by ventilation at room temperature whereupon a crude germ fraction was prepared.

After that, germs were selected from the crude germ fraction utilizing the difference in color as follows using a color selector of a belt type (BLM-300K; manufactured by K. K. Anzai Seisakusho; sold by K. K. Anzai Sogyo). This color selector is an apparatus having a means for irradiating the light to the crude germ fraction, a means for detecting reflected light and/or transmitted light from the crude germ fraction, a means for comparing the detected value with the standard value and a means for selecting and excluding those which were out of a standard value or within the standard value.

The crude germ fraction was supplied onto a belt in beige of the color selector so as to make it 1, 000 to 5, 000 grains/cm², light was irradiated onto the crude germ fraction on the belt using a fluorescent lamp and the reflected light was detected. Conveying speed of the belt was made 50 m/minute. With regard to a light-receiving sensor, a monochromatic CCD line sensor (2048 pixels) was used.

Firstly, in order to exclude black components (seed coats, etc.) from germs, the standard value was set between luminance of germs and luminance of seed coats and those which were out of the standard value were removed by suction. After that, in order to select the endosperm, the standard value was set between luminance of the germ and luminance of the endosperm and those which were out of the standard value were removed by suction. The suction was carried out using 30 suction nozzles (each suction nozzle was placed with interval of 1 cm of the length) placed at the position of about 1 cm above the conveying belt.

As a result of repetition of the above method, germs were selected until a purity of the germs (a rate by weight of germs contained in 1 g of any sample) reached 98% or more.

The resulting wheat germ fraction was suspended in distilled water of 4°C and washed using an ultrasonic washing machine until the washing was no longer turbid. After that, it was suspended in a 0.5% (by volume) solution of Nonidet P 40 (manufactured by Nakarai Techtonics) and washed using an ultrasonic washing machine until the washing was no longer turbid and resulting wheat germs were subj ected to the following operation at 4°C.

To wet weight of washed germs was added a 2-fold by volume of an extracting solvent (80 mM of HEPES-KOH (pH 7.8), 200 mM of potassium acetate, 10 mM of magnesium acetate and 8 mM of dithiothreitol (0.6 mM each of 20 kinds of L-amino acids may be added thereto)) and a limited disintegration of germs was carried out for three times using a Waring blender at 5, 000 to 20, 000 rpm for 30 seconds each. The homogenate was centrifuged at 30,000 × g for 30 minutes using a high-speed centrifugal separator, the resulting supernatant liquid was centrifuged again under the same condition, and the supernatant liquid was prepared. The sample did not show a decrease in its activity upon a long-term preservation at -80°C or lower. The resulting supernatant liquid was passed through a filter having a pore size of 0.2 µm (New Stelladisk 25 manufactured by Kurabo) whereupon filtration, sterilization and removal of contaminated fine dusts were conducted.

After that, the filtrate was subjected to the gel filtration using a Sephadex G-25 column which was previously equilibrated with a solution (40 mM of HEPES-KOH (pH 7.8), 0, 25, 50 or 100 mM of potassium acetate, 5 mM of magnesium acetate, 8 mM of dithiothreitol and 0.3 mM each of 20 kinds of L-amino acid mixed solution (depending upon the object for the synthesis of protein, the amino acids may not be added or they may be labeled amino acids)). The resulting filtrate was centrifuged again at 30,000 × g for 30 minutes and concentration of the recovered supernatant liquid was adjusted to an extent where A_{260 nm} was 90 to 150 (A₂₆₀/A₂₈₀ = from 1.4 to 1.6).

### (2) Making into the freeze-dried preparation

Cell extracts in which the deliquescent substance was decreased prepared in the above (1) (a potassium acetate concentration = 0 to 50 mM) and a conventional solution containing the cell extract containing 100 mM potassium acetate as a control were freeze-dried as follows.

Each solution containing the cell extract was frozen with liquid nitrogen and dehydrated by an operation for 3 hours using a freeze drying machine (Freeze Dry System Freezone 3.5 manufactured by Labconco) in accordance with the directions for use attached thereto. The prepared powdery sample was sealed in tubes by two kinds of methods - *in vacuo* and being filled with nitrogen - so that components therein were not chemically changed and then preserved at 4°C.

### Example 2. Analysis of protein synthesis activity of the freeze-dried preparation

To the freeze-dried preparation (corresponding to 50 µl of the cell extract) manufactured in Example 1 were added each 40 to 45 µl (in terms of the final concentration) of 20 mM of HEPES-KOH (pH 7.6), 100 mM of potassium acetate, 2.65 mM of magnesium acetate, 0.380 mM of spermidine (manufactured by Nakarai Techtonics), 0. 3 mM of each of 20 kinds of L-amino acids, 4 mM of dithiothreitol, 1.2 mM of ATP (manufactured by Wako Pure Chemicals), 0.25 mM of GTP (manufactured by Wako Pure Chemicals), 16 mM of creatine phosphate (manufactured by Wako Pure Chemicals), 1 U/µl of RNase Inhibitor (manufactured by Takara) and 0.5 µg/l of creatine kinase (manufactured by Roche)). After they were well dissolved, 1 µg of a translation template mRNA (ΩGFP) was placed therein and the reaction was carried out at 26°C for 24 hours and 48 hours in a dialysis system to a 10-fold by volume (to the reaction solution) of the outer liquid for the dialysis (20 mM of HEPES-KOH (pH 7.6), 100 mM of potassium acetate, 2.65 mM of magnesium acetate, 4 mM of dithiothreitol, 1.2 mM of ATP, 0.25 mM of GTP, 16 mM of creatine phosphate, 0.5 mg/ml of creatine kinase, 0.380 mM of spermidine, 20 kinds of L-amino acids (0.3 mM each) and 0.005% of NaN₃). As to the dialysis membrane, Spectra/Pore 6 having an exclusion molecular weight of 50,000 (Spectrum Laboratories Inc., CA, U. S. A.) was used. As a control, a solution containing the cell extract which was not freeze-dried was also subjected to the protein synthesis reaction in the same manner.

With regard to mRNA which is to be a translation template (ΩGFP), a transcription was carried out using SP6 RNApolymerase (manufactured by Toyobo) where an omega (Ω) sequence of tobacco mosaic virus (TMV) which is a translation initiation reaction sequence connected to SP6 promoter sequence and a plasmid GFP/pEU containing GFP gene DNA connected to 3'-downstream side (WO 01/27260) were templates and the resulting RNA was extracted with phenol/chloroform, precipitated with ethanol, purified by Nick Column (manufactured by Amersham Pharmacia Biotech) and used.

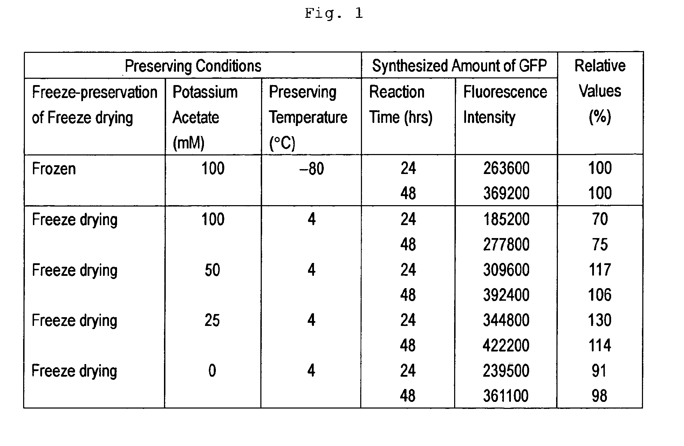

A GFP activity in the synthetic reaction solution after completion of the reaction was quantified from a fluorescence intensity using TD-360 Mini-Fluorometer manufactured by Turner Designs according to the means attached thereto. The result thereof is shown in Fig. 1. As will be apparent from Fig. 1, the freeze-dried preparation where the freeze drying was conducted in which the potassium acetate concentration in the solution containing the cell extract was 100 mM showed a decrease in the protein synthesis activity to an extent of about seven tenth as compared with that which was not freeze-dried. However, in the product where the freeze drying was conducted where the potassium acetate concentration was not more than 50 mM, the activity was found to be same or rather increased as compared with a product which was not freeze-dried even after the reaction was conducted for 48 hours.

### Example 3. Analysis of protein synthesis activity in case the solution containing the cell extract wherefrom the low-molecular inhibitor for the protein synthesis was excluded was made into a freeze-dried product

### (1) Exclusion of the low-molecular inhibitor for the protein synthesis using the dialysis membrane

The wheat germ extract prepared in Example 1 contained factors necessary for protein synthesis (tRNA, aminoacyl tRNA synthase, ribosome, translation initiation factor, peptide chain elongation factor, translation termination factor, etc.) and other molecules, particularly a substance which specifically or non-specifically inhibits the synthesis of protein. Therefore, it was fractionated utilizing the difference in molecular weights whereupon the wheat extract wherefrom protein inhibiting component was excluded was prepared (Japanese Patent Application No. 2002/023,141).

The dialysis membrane was used for a fractionation. As to the dialysis membrane, Spectra/Pore 6 having an exclusion molecular weight of 50,000 (manufactured by Spectrum Laboratories Inc., CA, U. S. A.) was used. The exclusion molecular weight was selected in such a sense that which does not pass the factor necessary for the protein synthesis and is able to fractionate molecules having the molecular weight less than that.

With regard to the wheat germ extract which was subj ected to dialysis, that to which 2/3 volume (of the total volume) of the wheat germ extract (prepared in Example 1), 30 mM of HEPES-KOH (pH 7.6), 0/50/100 mM of potassium acetate, 2.65 mM of magnesium acetate, 2.85 mM of dithiothreitol, 0.380 mM of spermidine, 1,000 units/ml of ribonuclease inhibitor (RNase inhibitor), 16 mM of creatine phosphate, 0. 5 mg/ml of creatine kinase and 1.2 mM of ATP were added was prepared and used.

The dialysis was carried out by being allowed to stand at 26°C or 4°C for 12 hours using each of the above-mentioned extracts and a solution of the same composition except that contained no germ extract the outer liquid for the dialysis.

A part of the inner liquid for the dialysis was freeze-dried by the same manner as in Example 1 while the remaining part was preserved at -80°C. With regard to the preparation after being freeze-dried, the potassium acetate solution was added so that the final concentration of potassium acetate became 100 mM. With regard to the protein synthesis reaction, it was carried out at 26°C for 24 hours and 48 hours where 1 µg of the translation template mRNA (ΩGFP) was placed in each of the above-mentioned reaction solutions and in the dialysis system using a 10-fold by volume (to the reaction solution) of the outer liquid for the dialysis (20 mM of HEPES-KOH (pH 7.6), 100 mM of potassium acetate, 2.65 mM of magnesium acetate, 4 mM of dithiothreitol, 1.2 mM of ATP, 0.25 mM of GTP, 16 mM of creatine phosphate, 0.5 mg/ml of creatine kinase, 0.380 mM of spermidine, 20 kinds of L-amino acids (0.3 mM each) and 0.005% of NaN₃). As to the dialysis membrane, Spectra/Pore 6 having the exclusion molecular weight of 50, 000 (manufactured by Spectrum Laboratories Inc., CA, U. S. A.) was used.

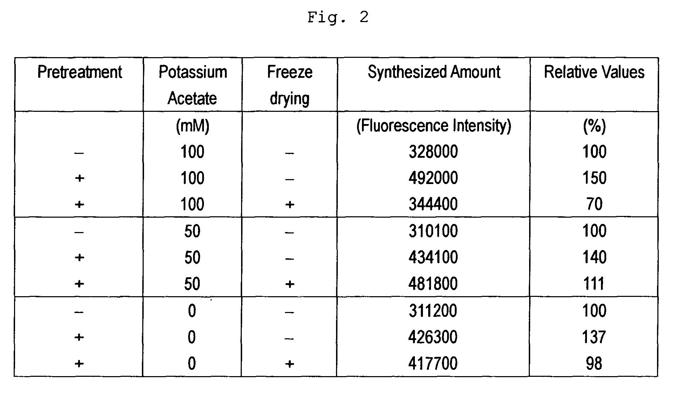

The GFP activity in the synthesis reaction solution after completion of the reaction was quantified from the fluorescence intensity using TD-360 Mini-Fluorometer manufactured by Turner Designs according to the means attached thereto. The result thereof is shown in Fig. 2. In the drawing, a pretreatment means an exclusion treatment of the low-molecular inhibitor for the protein synthesis which was conducted above, "-" means that the solution containing the cell extract which was not subjected to said treatment was used and "+" means that the solution containing the cell extract which was subjected to said treatment was used. Similarly, with regard to the freeze drying, "-" means that a solution preserved at -80°C was used and "-" means that a product made into a freeze-dried preparation was used. As will be apparent from Fig. 2, when the freeze drying is conducted where the potassium acetate concentration is 100 mM, protein synthesis activity is inferior to a substance preserved at low temperature even in the case of the solution containing the cell extract wherefrom the low-molecular inhibitor for the protein synthesis is excluded. On the contrary, a product where the potassium acetate concentration is made not more than 50 mM after the exclusion treatment for the low-molecular inhibitor for the protein synthesis has the same or even more protein synthesis activity as compared with the product which was preserved at low temperature.

### Industrial Applicability

The freeze-dried preparation for the cell-free protein synthesis according to the present invention and the method for the synthesis of protein using the same do not require preservation at extremely low temperature and troublesome operations which have been necessary in the conventional reagents for the cell-free protein synthesis reaction and are able to be important tools for construction of a fully-automated system for the high-throughput protein synthesis.

## Claims

1. A method for a manufacture of a freeze-dried preparation for a cell-free protein synthesis by a freeze drying of a solution containing a cell extract for a cell-free protein synthesis, wherein a content of a deliquescent substance contained in the solution is in such an amount that it does not affect a quality of a preparation after being freeze-dried.

2. A freeze-dried preparation for the cell-free protein synthesis according to claim 1, wherein the amount of the deliquescent substance is not more than 0.01 part by weight to 1 part by weight of the protein in the dried preparation.

3. The method according to claim 1 or 2, wherein the deliquescent substance is selected from potassium acetate, magnesium acetate and calcium acetate.

4. The method according to claim 1, wherein the method includes a step where a low-molecular inhibitor for the protein synthesis is excluded from the solution containing the cell extract for the cell-free protein synthesis.

5. The method according to claim 4, wherein the low-molecular inhibitor for the protein synthesis has a molecular weight of not more than 14,000 daltons.

6. The method according to claim 4 or 5, wherein exclusion of the inhibitor for the protein synthesis is carried out in a solution which at least contains a high-energy phosphoric acid compound.

7. The method according to any of claims 1 to 6, wherein the solution containing a cell extract for a cell-free protein synthesis is a wheat germ extract.

8. The method according to any of claims 1 to 7, wherein the solution containing the cell extract for the cell-free protein synthesis is that to which a solution containing amino acid which is a substance necessary for the protein synthesis at least acting as a substrate therefor and energy source are contained is added.

9. A freeze-dried preparation for a cell-free protein synthesis which is manufactured by the method mentioned in claims 1 to 8.

10. A kit for a cell-free protein synthesis containing the preparation mentioned in claim 9.

11. A kit for a cell-free protein synthesis in which the preparation mentioned in claim 9 is combined with a solution so that concentration a solution of at least the deliquescent substance is adjusted to optimum during the cell-free protein synthesis.

12. The kit for the protein synthesis according to claim 10 or 11, wherein a protein library is able to be synthesized by way of containing a translation template.

13. A method for the protein synthesis in which a solution containing a cell extract for a cell-free protein synthesis which is prepared using the kit for the cell-free protein synthesis mentioned in claims 10 to 12 is used.

14. The method for the protein synthesis according to claim 13, wherein a preparation is conducted so as to make a final concentration of potassium acetate 50 to 200 mM.

15. A synthetic protein which is prepared by the method for the protein synthesis mentioned in claim 13.
